# EUROPEAN PATENT SPECIFICATION

(11) **EP 2 723 702 B1**
(45) Date of publication and mention of the grant of the patent: **14.06.2017**
(21) Application number: 12804027.6
(22) Date of filing: 24.05.2012
(51) Int. Cl.: C07C 2/64, C07C 15/085, B01J 19/26, C07C 7/04, C07C 2/66

(54) **EXCHANGER AND A PROCESS RELATING THERETO**
WÄRMETAUSCHER UND ZUGEHÖRIGES VERFAHREN
ÉCHANGEUR ET PROCÉDÉ ASSOCIÉ

(30) Priority: 27.06.2011 US 201113170143
(43) Date of publication of application: 30.04.2014
(73) Proprietor: UOP LLC, Des Plaines, Illinois 60017-5017 (US)
(72) Inventor: SMITH, Michael Roy, Des Plaines, Illinois 60017-5017 (US); SCHULZ, Russell, Des Plaines, Illinois 60017-5017 (US); DALY, Phillip F., Des Plaines, Illinois 60017-5017 (US); SECHRIST, Paul Alvin, Des Plaines, Illinois 60017-5017 (US)
(74) Representative: Boult Wade Tennant
(86) International application number: PCT/US2012/039249
(87) International publication number: WO 2013/002923

(56) References cited:
- WO-A1-2009/088511
- KR-A- 20100 123 778
- KR-B1- 101 007 481
- US-A- 4 555 311

## Description

### STATEMENT OF PRIORITY

This application claims priority to U.S. Application No. 13/170,143 which was filed on June 27, 2011.

### FIELD OF THE INVENTION

This invention generally relates to processes, such as alkylation, utilizing one or more exchangers.

### DESCRIPTION OF THE RELATED ART

WO 2009/088511 describes a process for separating diisopropylbenzene (DIPB) and triisopropylbenzene (TIPB) from a feed comprising DIPB, TIPB and polyalkylate heavies.

US 4,555,311 describes an integrated fractionation in the recovery of alkylaromatic hydrocarbons.

The alkylation of benzene with propene may be conducted in a multi-stage, all liquid phase reactor under conditions sufficient to limit reaction temperature rise. Often, controlling the reaction temperature may be obtained by the staged addition of propene reactant also serving as an inter-stage reactor quench with a large circulating reactor effluent recycle stream passing from the final reaction stage back to the first reaction stage. The reactor effluent recycle stream can act as a heat sink to control the exotherm in the multiple stages.

As an example, the amount of the reactor effluent recycle stream can be 3.5 to 1, by volume, based on the net reactor effluent. As such, the reactor effluent recycle stream may require the use of large-capacity recycle pumps combined with various heat exchange services that cool a portion of the recycle stream before providing the inter-bed reactor quench. As a consequence, there is a strong desire to improve the efficiency of the process by utilizing heat generated from the stages rather than merely utilizing cooling apparatuses without the benefit of recapturing the heat in other suitable processes.

### SUMMARY OF THE INVENTION

One exemplary embodiment can be a process for alkylating. The process can include providing a first effluent from a first alkylation zone, and providing a second effluent from a second alkylation zone. Generally, the first and second effluents are provided to an exchanger for reboiling a fractionation zone.

The exchanger can include a first discrete tube bundle, a second discrete tube bundle, and a third discrete tube bundle. Typically, each tube bundle is adapted to receive a different stream for exchanging heat.

A further exemplary embodiment may be a process for alkylating benzene with one or more C3- hydrocarbons. The process can include providing a first effluent including one or more alkylated benzene compounds from a first alkylation zone, providing a second effluent including one or more alkylated benzene compounds from a second alkylation zone, providing a third effluent including one or more alkylated benzene compounds from a third alkylation zone, passing the first, second, and third effluents from the stab-in reboiler to a cooling zone including one or more air coolers, and providing the first effluent to the second alkylation zone, the second effluent to the third alkylation zone, and the third effluent to a fourth alkylation zone from the cooling zone. Usually, the first, second, and third effluents are provided to a stab-in reboiler for intermediate reboiling of a benzene fractionation column.

The embodiments disclosed herein can provide a process that utilizes a plurality of effluents, namely a first and second effluent from respective first and second alkylation zones to provide heat in another process, such as reboiling a fractionation zone. Preferably, at least three effluents from respective alkylation zones are used for reboiling a fractionation zone, such as a fractionation zone separating cumene and benzene where the exchanger can be a stab-in reboiler. Utilizing an intermediate reboiler located higher in the column where the temperature differential driving forces are favorable can decrease the use of higher value, hotter heating medium, such as heat transfer oil, high temperature steam or a fuel-fired heater at the bottom reboiler, thereby improving energy efficiency and reducing external utility costs. In addition, the embodiments disclosed herein can provide an exchanger with three discrete tube bundles, each having a separate inlet and outlet, that can receive separate process streams preferably all being at substantially the same temperature for providing additional heat duty to, e.g., a fractionation zone. Moreover, eliminating the inter-bed quench can improve mono-alkylation selectivity from a plug flow reactor configuration versus backmixing that may occur from an inter-bed quench.

### DEFINITIONS

As used herein, the term "stream" can include various hydrocarbon molecules, such as straight-chain, branched, or cyclic alkanes, alkenes, alkadienes, and alkynes, and optionally other substances, such as gases, e.g., hydrogen, or impurities, such as heavy metals, and sulfur and nitrogen compounds. The stream can also include aromatic and nonaromatic hydrocarbons. Moreover, the hydrocarbon molecules may be abbreviated C1, C2, C3...Cn where "n" represents the number of carbon atoms in the one or more hydrocarbon molecules.

As used herein, the term "zone" can refer to an area including one or more equipment items and/or one or more sub-zones. Equipment items can include one or more reactors or reactor vessels, heaters, exchangers, pipes, pumps, compressors, and controllers. Additionally, an equipment item, such as a reactor, dryer, or vessel, can further include one or more zones or sub-zones.

As used herein, the term "alkylation" can mean the introduction of an alkyl radical into an organic molecule. As an example, an ethene and/or a propene radical can be introduced into a benzene molecule or a methanol radical can be introduced into a toluene molecule.

As used herein, the term "exchanger" may refer to an equipment item that facilitates heat transfer between two fluids and can include a stab-in reboiler, a condenser, an evaporator, an air cooler, a kettle exchanger, a double-piped exchanger, a baffled shell-and-tube exchanger, a forced-recirculation reboiler, or a vertical or horizontal thermosiphon reboiler.

As used herein, the term "rich" can mean an amount of at least generally 50%, and preferably 70%, by mole, of a compound or class of compounds in a stream.

As used herein, the term "substantially" can mean an amount of at least generally 80%, preferably 90%, and optimally 99%, by mole, of a compound or class of compounds in a stream.

As depicted, process flow lines in the figures can be referred to interchangeably as, e.g., lines, pipes, feeds, portions, effluents, remainders, products, or streams.

### BRIEF DESCRIPTION OF THE DRAWINGS

FIG. 1 is a schematic depiction of an exemplary alkylation apparatus.
FIG. 2A is an elevational, end view of an exemplary reboiler along lines 2A-2D of FIG. 1.
FIG. 2B is an elevational, end view of another exemplary reboiler along lines 2A-2D of FIG. 1.
FIG. 2C is an elevational, end view of a further exemplary reboiler along lines 2A-2D of FIG. 1.
FIG. 2D is an elevational, end view of yet another exemplary reboiler along lines 2A-2D of FIG. 1.

### DETAILED DESCRIPTION

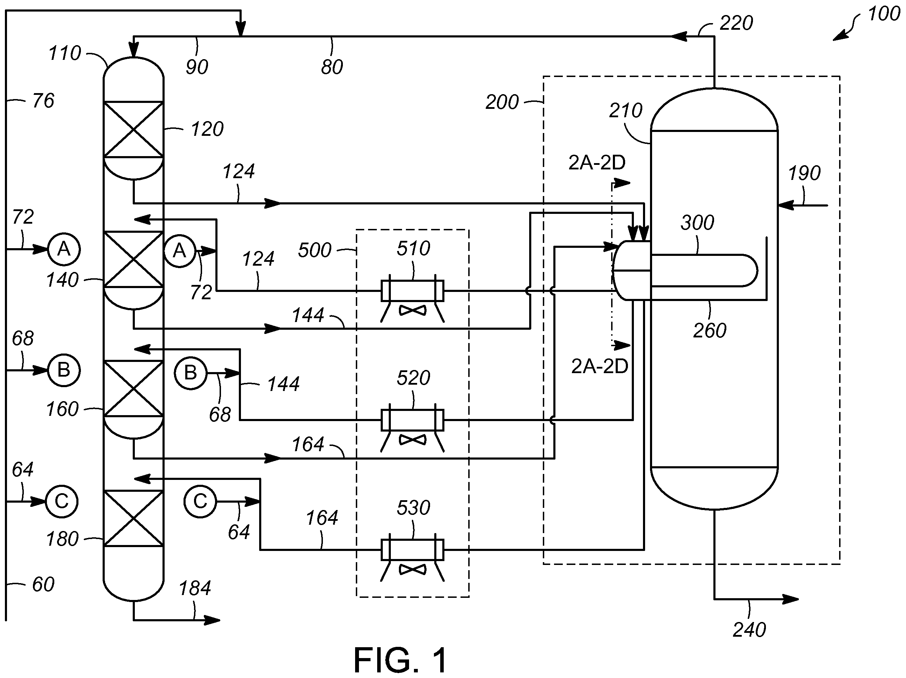

Referring to FIG. 1, an alkylation apparatus 100 can include an alkylation reactor 110, a fractionation zone 200 and a cooling zone 500. Usually, the cooling zone 500 is provided if additional heat removal is required before returning an effluent to an alkylation zone, as hereinafter described. Generally, the alkylation apparatus 100 can receive a feed 60 including one or more C3 hydrocarbons, typically propene, that may be split into several streams. Particularly, the C3 feed 60 can be split into a first propene portion 64, a second propene portion 68, and a third propene portion 72, that can be provided directly to recycled effluents, respectively at "C", "B", and "A" as depicted in FIG. 1 and are discussed in further detail hereinafter. The propene remainder stream 76 can be combined with a recycle stream 80, including benzene, to form a combined feed 90. The recycle stream 80 will be discussed in further detail hereinafter.

The alkylation reactor 110 can include a first alkylation zone 120, a second alkylation zone 140, a third alkylation zone 160, and a fourth alkylation zone 180. Although four alkylation zones are disclosed, it should be understood that any suitable number of alkylation zones may be utilized such as two to eight alkylation zones. The first, second, third and fourth alkylation zones 120, 140, 160, and 180 can operate at any suitable conditions, independently, such as a temperature of 70 - 180°C and a pressure of 100 - 10,000 kPa. The alkylation zone effluent 184 can be passed for further processing, such as to a fractionation zone to remove light ends, which may include a depropanizer column, and be combined with other process effluents, such as a transalkylation effluent prior to being further fractionated. These exemplary zones are disclosed in, e.g., US 7,744,828. Additionally, if the cooling zone 500 is insufficient to remove heat from alkylation effluent, at least a portion of the alkylation zone effluent 184 may be recycled to the top of the alkylation reactor 110 by being added to the combined feed 90, as hereinafter described. Generally, it is desirable to minimize any such recycling of the alkylation zone effluent 184.

After additional processing, the alkylation zone effluent 184 can be provided whole or in part as a benzene column feed 190. Particularly, the fractionation zone 200 can include a fractionation column 210, such as a benzene column. The fractionation column 210 can include a tub 260 housing at least a portion of an exchanger 300. The exchanger 300 in this exemplary embodiment can be a stab-in reboiler to provide intermediate heat duty to the fractionation column 210.

Usually, the fractionation column 210 can provide an overhead stream 220, including benzene, and a bottom stream 240, including an alkylated benzene such as cumene. The fractionation column 210 can operate at any suitable conditions, such as an operating temperature ranging from 30 - 220° C and a pressure ranging from 160 - 280 kPa. The benzene in the overhead stream 220 can be provided at a temperature of 120 - 170° C. The overhead stream 220 can be combined with the propene remainder stream 76 to form the combined feed 90 to the alkylation reactor 110.

In operation, the combined feed 90 can be provided to the first alkylation zone 120 forming a first effluent or first alkylation effluent 124. The first effluent 124 can be provided to the exchanger 300 to remove some of its heat. Generally, liquid in the fractionation column 210 collects in the tub 260, which can be reboiled by the exchanger 300. Afterwards, the first effluent 124 can be provided to the cooling zone 500.

The cooling zone 500 can include one or more air coolers, such as a first air cooler 510, a second air cooler 520, and a third air cooler 530, although any suitable number of coolers can be utilized. Also, the cooler may utilize other fluids, such as water, in one preferred embodiment the cooler is one or more air-cooled heat exchangers. Alternatively, any suitable exchanger can be utilized. Afterwards, the first effluent 124 can pass through the cooling zone 500 particularly, the first air cooler 510 to be cooled to a suitable temperature for being provided to the second alkylation zone 140. Prior to being provided above the second alkylation zone 140, the first effluent 124 can be combined with the third propene portion 72.

Similarly, a second effluent or second alkylation effluent 144 and a third effluent or third alkylation effluent 164 can exit the respective second and third alkylation zones 140 and 160. Likewise, the second effluent 144 and the third effluent 164 may be provided to the exchanger 300. Afterwards, the second effluent 144 and the third effluent 164 can pass through the cooling zone 500, particularly the respective second air cooler 520 and third air cooler 530, to be cooled to the appropriate temperature prior to being combined with, respectively, the second propene portion 68 and the first propene portion 64. Next, the second effluent 144 and the third effluent 164 can be provided to the above-respective alkylation zones, namely the third alkylation zone 160 and the fourth alkylation zone 180. The alkylation zone effluent 184 can be obtained after the reactants pass through the fourth alkylation zone 180. The cooling zone 500 can provide a cooled recycled effluent to an alkylation zone for preventing an undesirable increase in the exothermic reaction temperature.

Typically, the effluents 124, 144, and 164 can be transferred as a liquid under system pressure at an intermediate location between the benzene column feed 190 and bottom stream 240 of the benzene column 210. Internal reactor heads can ensure full flow of the effluents 124, 144, and 164 to the exchanger 300 and back to the alkylation reactor 110. After transferring heat to the material in the benzene column 210, the cooled bed effluents 124, 144, 164 return, separately, to the reactor immediately downstream of the original take-up points after cooling and mixing with more propene-rich reactant.

Usually, the first effluent 124, the second effluent 144, and the third effluent 164 are provided to the exchanger 300 at substantially the same pressure and temperature. As such, mechanical stress on the exchanger components due to temperature and pressure differentials is minimized. The exchanger disclosed herein may only have a tube side temperature difference of 10° C while in service from the inlet to the outlet. Thus, the low temperature difference generally allows the use the three or more discrete tube bundles while preventing damage due to fatigue.

The exchanger 300 can include a single stab-in reboiler partitioned into discrete bundles for separate streams to pass through the exchanger 300 without co-mingling. Usually, there is a discrete tube bundle for each process stream, and in this exemplary embodiment there are three discrete tube bundles. Although a stab-in reboiler is disclosed, it should be understood that other types of exchangers could be utilized such as a kettle exchanger or a horizontal thermosiphon reboiler. The kettle exchanger and the thermosiphon reboiler may be external to the fractionation column 210. An external exchanger may be advantageous if a stab-in reboiler is not suitable for the fractionation column 210 due to mechanical constraints.

Thus, the embodiment disclosed herein can allow the transfer of a portion of the heated reacted effluents from three separate zones within the alkylation reactor 110 to a location within the alkylation apparatus 100 to effectively reduce the use of higher value, hotter heating medium, such as heat transfer oil, high temperature steam or a fuel-fired heater for the generation of recycled benzene required for the alkylation reactor 110. In one exemplary embodiment, a single three-in-one service heat exchanger utilizing three discrete bundles can obtain these results. Typically, a discrete tube bundle isolates the fluid therein from other fluids passing through the exchanger.

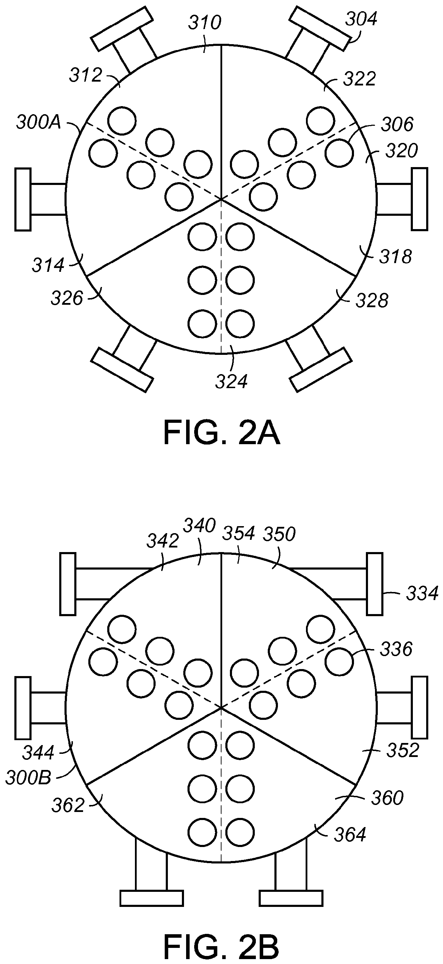

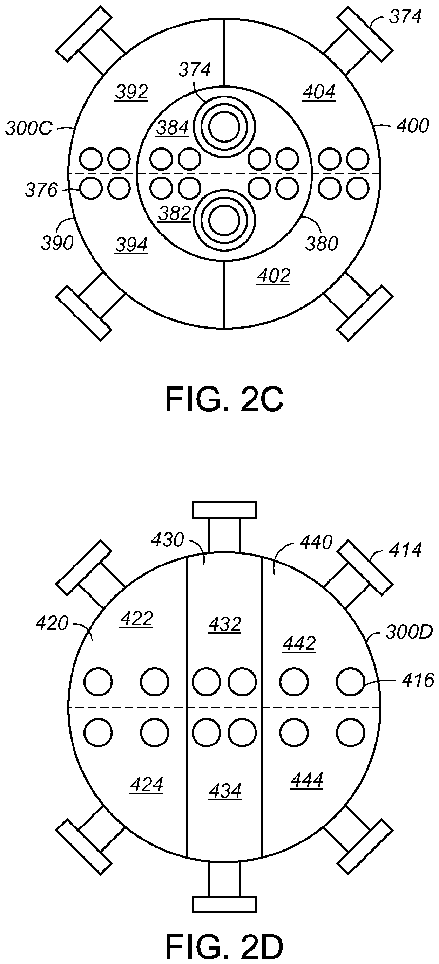

Referring to FIGS. 2A-2D, the exchanger 300 can take several forms. Particularly, the exchanger 300 can be a reboiler 300A as depicted in FIG. 2A, a reboiler 300B as depicted in FIG. 2B, a reboiler 300C as depicted in FIG. 2C, or a reboiler 300D as depicted in FIG. 2D. Each of these reboilers 300A-D can have three discrete tube bundles, as depicted, with an internal position depicted by one or more dashed lines.

Referring to FIG. 2A, the reboiler 300A can include one or more nozzles 304 and one or more U-tubes 306. Although six nozzles 304 are depicted and nine U-tubes are depicted, it should be understood that only one nozzle 304 and one U-tube 306 are numbered so as to not unduly clutter the drawing. Moreover, only some of the U-tubes 306 are depicted although other U-tubes may also be present. Likewise, the other FIGS. 2B-D may be similarly numbered and may only depict some of the U-tubes.

The reboiler 300A can include a first sector 310, with an inlet 312 and an outlet 314, a second sector 318 with an inlet 320 and an outlet 322, and a third sector 324 with an inlet 326 and an outlet 328. Each sector 310, 318, and 324 can be internally partitioned to segregate the inlets and outlets as indicated by the dashed lines. In this exemplary embodiment, the nozzles 304 are orientated substantially perpendicular to a tangent line passing through a point on the arc where a line passing through the point and center of the exchanger bisects an inlet pie-shape, e.g., inlet 312, or an outlet pie-shape, e.g., outlet 314, as viewed from an end. Dimensionally, each sector 310, 318, and 324 can define a portion of a circle enclosed by two radii and an arc, as viewed from an end of the reboiler 300A. Generally, each sector 310, 318, and 324 supports a discrete set of tube bundles that can receive, independently, a process stream. As an example, a process fluid can enter the inlet 312 and exit the outlet 314 of the first sector 310. In this instance, each sector 310, 318, and 324 may contain discrete tube bundles and can receive a respective alkylation effluent, namely the first alkylation effluent 124, the second alkylation effluent 144, and the third alkylation effluent 164 as depicted in FIG. 1.

Referring to FIG. 2B, the exemplary reboiler 300B is depicted. Generally, the reboiler 300B can include one or more nozzles 334 and one or more U-tubes 336. The nozzles 334 can be provided at any suitable orientation to support piping and to allow ease of maintenance inside the reboiler 300B. In this instance, some of the nozzles 334 can be at a different orientation than the nozzles 304 of the reboiler 300A. In the depicted example, four of the nozzles 334 can be offset from a perpendicular to a tangent line passing through a point on the arc where a line passing through the point and center of the exchanger bisects an inlet pie-shape, e.g., inlet 342, or an outlet pie-shape, e.g., outlet 344. Some of the nozzles 334, in this case two nozzles, can be orientated the same as the nozzles 304 of the reboiler 300A. The reboiler 300B, can have a first sector 340 having an inlet 342 and an outlet 344, a second sector 350 having an inlet 352 and an outlet 354, and a third sector 360 having an inlet 362 and an outlet 364, similar to the reboiler 300A. The reboiler 300B can receive effluents similarly as the reboiler 300A.

Referring to FIG. 2C, the reboiler 300C can include one or more nozzles 374 and one or more U-tubes 376. Similar to the versions described above, the nozzles 374 are orientated at various angles to support piping and to allow ease of maintenance. The reboiler 300C can form a concentric circular region 380 having an inlet 382 and an outlet 384, a first semi-arcuate segment 390 having an inlet 392 and an outlet 394, and a second semi-arcuate segment 400 having an inlet 402 and an outlet 404. Collectively the first and second semi-arcuate segments 390 and 400, and the concentric circular region 380 may form concentric circles, as viewed from an end of the reboiler 300C. Generally, the concentric circular region 380, and the first and second semi-arcuate segments 390 and 400 may each contain a discrete tube bundle and can receive, respectively, the first, second, and third alkylation effluents 124, 144, and 164.

Referring to FIG. 2D, the exemplary reboiler 300D can include one or more nozzles 414 and one or more U-tubes 416. Generally, the reboiler 300D can include a first segment 420 having an inlet 422 and an outlet 424, a second segment 430 having an inlet 432 and an outlet 434, and a third segment 440 having an inlet 442 and an outlet 444. Each segment 420, 430, or 440 can define an area separated from the rest of the circle by one or two chords, as viewed at an end of the reboiler 300D. Each segment 420, 430, and 440 may contain a discrete tube bundle and can receive a respective alkylation effluent, namely the first alkylation effluent 124, the second alkylation effluent 144, and the third alkylation effluent 164.

Usually, each one of these reboilers 300A-D provides a partitioned exchanger having separate sectors, segments, and/or regions that allow the reception of three separate process fluids into three discrete tube bundles. Although the tubes are disclosed as being U-tubes, it should be understood that any suitable tubes can be utilized, such as straight tubes with a floating tube sheet and a suitably partitioned return head. Moreover, the placement of nozzles is merely exemplary and may be provided in any suitable position to provide support for piping and ease of access during, e.g., maintenance of the exchanger, such as a reboiler. In many instances, the inlet temperature conditions and the heat removal loads are almost identical for the process streams passing through the exchanger. As such, the partitioning of the bundle is essentially for equal surface requirements.

The tube sheet can be segmented into sections, segments, or regions of approximately equal surface area and the number of tubes. The cover of the exchanger can be similarly partitioned to match so the process streams may be kept separated during introduction and removal from a bundle. Each partitioned portion of the exchanger can have its own internal partition for internal inlet and return flow. The internal partition for each partitioned portion is depicted by dashed lines in FIGS. 2A-D.

To minimize heat transfer surface requirements, the tubes can be modified with surface heat transfer enhancements. Such enhancements can include one or more external fins, internal ridges, and/or porous layers. Procedures for making fins, grooves and/or ridges inside and/or outside a tube are disclosed in, e.g., US 2,181,927, US 3,559,437, US 3,847,212, and US 2005/0145377 A1. Moreover, the inside and/or outside of the tubes can be coated, e.g., with a porous layer, such as that disclosed in, e.g., US 4,064,914 and US 5,091,075, to improve heat transfer and to allow boiling at a much closer temperature approach for lower hot side inlet and/or outlet temperatures. Desirably, the bundles' overall tube sheet diameter is as small as possible to minimize the size of the stab-in bundle and bundle support system requirements to minimize capital costs and to allow the fitting of a stab-in reboiler into a fractionation column. Using tubes of smaller diameter can also improve tube side heat transfer performance. Although three discrete tube bundles are disclosed, exchangers with more than three discrete tube bundles can be utilized, such as four discrete tube bundles.

In one exemplary embodiment, a staggered arrangement of tube bundles compared to a flat arrangement of tube bundles may provide a greater utilization of space. As an example, having three separate tube bundles aligned in the same plane may take more space than having the middle tube bundle elevated at an angle up to 60°, preferably 30 - 60°, with respect to the level of the two outer tube bundles with the angle determined by the relative diameters of the bundles in a column.

The various nozzle arrangements as depicted in FIGS. 2A-D minimize interference between the nozzles. Optionally, the flanges of the nozzles may be in different planes. The thickness of the partitions and design of gaskets fitted to the partitions can depend on the differential pressures of the exchanger.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

In the foregoing, all temperatures are set forth in degrees Celsius and, all parts and percentages are by weight, unless otherwise indicated.

## Claims

1. A process for alkylating, comprising:
A) providing a first effluent from a first alkylation zone; and
B) providing a second effluent from a second alkylation zone; wherein the first and second effluents are provided to an exchanger for reboiling a fractionation zone.

2. The process according to claim 1, further comprising providing a third effluent from a third alkylation zone to the exchanger.

3. The process according to claim 1 or 2, wherein the process is for alkylating benzene with one or more C3- hydrocarbons.

4. The process according to claim 3, wherein the one or more C3- hydrocarbons comprises propene.

5. The process according to claim 2, further comprising providing the first effluent to the second alkylation zone, the second effluent to the third alkylation zone, and the third effluent to a fourth alkylation zone after exiting the exchanger.

6. The process according to claim 2, wherein the first, second, and third effluents are provided to the exchanger for intermediate reboiling of the fractionation zone.

7. The process according to claim 1 or 2, wherein the exchanger is a stab-in reboiler.

8. The process according to claim 1 or 2, wherein the fractionation zone further comprises a fractionation column for separating benzene from alkylated benzene.

9. The process according to claim 8, wherein the alkylated benzene comprises cumene.

10. The process according to claim 2 or 5, further comprising passing the first, second, and third effluents through a cooling zone before providing to, respectively, the second alkylation zone, the third alkylation zone, and a fourth alkylation zone.

## Patentansprüche

1. Verfahren zum Alkylieren, umfassend:
A) Bereitstellen eines ersten Ausflusses aus einer ersten Alkylierungszone; und
B) Bereitstellen eines zweiten Ausflusses aus einer zweiten Alkylierungszone, wobei der erste und der zweite Ausfluss einem Wärmetauscher zum Verdampfen in einer Fraktionierungszone bereitgestellt werden.

2. Verfahren nach Anspruch 1, ferner umfassend Bereitstellen eines dritten Ausflusses aus einer dritten Alkylierungszone an den Wärmetauscher.

3. Verfahren nach Anspruch 1 oder 2, wobei das Verfahren zur Alkylierung von Benzol mit einem oder mehreren C₃⁻-Kohlenwasserstoffen dient.

4. Verfahren nach Anspruch 3, wobei der eine oder die mehreren C₃⁻-Kohlenwasserstoffe Propen umfassen.

5. Verfahren nach Anspruch 2, ferner umfassend Bereitstellen des ersten Ausflusses an die zweite Alkylierungszone, des zweiten Ausflusses an die dritte Alkylierungszone und des dritten Ausflusses an eine vierte Alkylierungszone nach Verlassen des Wärmetauschers.

6. Verfahren nach Anspruch 2, wobei der erste, der zweite und der dritte Ausfluss dem Wärmetauscher zum Zwischenverdampfen in der Fraktionierungszone bereitgestellt werden.

7. Verfahren nach Anspruch 1 oder 2, bei dem der Wärmetauscher ein innenliegender Reboiler ist.

8. Verfahren nach Anspruch 1 oder 2, wobei die Fraktionierungszone ferner eine Fraktionierkolonne zum Trennen von Benzol von alkyliertem Benzol umfasst.

9. Verfahren nach Anspruch 8, wobei das alkylierte Benzol Cumol umfasst.

10. Verfahren nach Anspruch 2 oder 5, ferner umfassend das Leiten des ersten, des zweiten und des dritten Ausflusses durch eine Kühlzone, bevor sie der zweiten Alkylierungszone, der dritten Alkylierungszone beziehungsweise einer vierten Alkylierungszone bereitgestellt werden.

## Revendications

1. Procédé d'alkylation, comprenant :
A) fourniture d'un premier effluent provenant d'une première zone d'alkylation ; et
B) fourniture d'un deuxième effluent provenant d'une deuxième zone d'alkylation ; dans lequel les premier et deuxième effluents sont fournis à un échangeur pour le rebouillage d'une zone de fractionnement.

2. Procédé selon la revendication 1, comprenant en outre la fourniture d'un troisième effluent provenant d'une troisième zone d'alkylation à l'échangeur.

3. Procédé selon la revendication 1 ou 2, le procédé étant destiné à l'alkylation de benzène avec un ou plusieurs hydrocarbures en C3.

4. Procédé selon la revendication 3, dans lequel les un ou plusieurs hydrocarbures en C3 comprennent le propène.

5. Procédé selon la revendication 2, comprenant en outre la fourniture du premier effluent à la deuxième zone d'alkylation, du deuxième effluent à la troisième zone d'alkylation, et le troisième effluent à une quatrième zone d'alkylation après la sortie de l'échangeur.

6. Procédé selon la revendication 2, dans lequel les premier, deuxième, et troisième effluents sont fournis à l'échangeur pour rebouillage intermédiaire de la zone de fractionnement.

7. Procédé selon la revendication 1 ou 2, dans lequel l'échangeur est un rebouilleur à guidage intérieur.

8. Procédé selon la revendication 1 ou 2, dans lequel la zone de fractionnement comprend en outre une colonne de fractionnement pour séparer le benzène du benzène alkylé.

9. Procédé selon la revendication 8, dans lequel le benzène alkylé comprend du cumène.

10. Procédé selon la revendication 2 ou 5, comprenant en outre le passage des premier, deuxième et troisième effluents à travers une zone de refroidissement avant fourniture à, respectivement, la deuxième zone d'alkylation, la troisième zone d'alkylation, et une quatrième zone d'alkylation.
